Europäisches Patentamt

European Patent Office

Office européen des brevets

(19)

(11) Veröffentlichungsnummer : **0 082 242**
**B1**

(12)                    EUROPÄISCHE PATENTSCHRIFT

(45) Veröffentlichungstag der Patentschrift :
28.05.86

(51) Int. Cl.⁴ : **A 61 B 19/00**

(21) Anmeldenummer : **82104809.7**

(22) Anmeldetag : **02.06.82**

(54) Geräteaufnahmesystem der Medizintechnik.

(30) Priorität : **14.11.81 DE 3145310**

(43) Veröffentlichungstag der Anmeldung :
**29.06.83 Patentblatt 83/26**

(45) Bekanntmachung des Hinweises auf die Patenterteilung : **28.05.86 Patentblatt 86/22**

(84) Benannte Vertragsstaaten :
**BE FR NL**

(56) Entgegenhaltungen :
**EP-A- 0 047 958**
**DE-B- 1 541 166**
**DE-B- 2 812 074**
**US-A- 3 504 386**

(73) Patentinhaber : **Drägerwerk Aktiengesellschaft
Moislinger Allee 53-55
D-2400 Lübeck 1 (DE)**

(72) Erfinder : **Thometschek, Roderich
Mozartstrasse 27
D-2406 Stockelsdorf (DE)**

# Beschreibung

Die Erfindung betrifft ein Geräteaufnahmesystem der Medizintechnik mit Einrichtungen zur Verriegelten Aufnahme vom Gerät am Einsatzort sowie zum verriegelten Transport.

In der Medizintechnik steigen am Ort des Einsatzes, sei es im OP-Bereich, in Untersuchungsräumen oder am Bett die Anforderungen nach mehr Geräten und Ausrüstungen immer weiter. Die vielartigen Geräte sind dabei zu Systemen zusammenzufassen und anzupassen. Dazu ist es notwendig, die Einheiten sicher zu transportieren und am Ort des Einsatzes einfach zu montieren bzw. einzuhängen. Es muß darüberhinaus gesichert sein, daß z. B. die Narkose- und Beatmungsgeräte von ihrem Einsatzort zur Reinigung und Überprüfung problemlos transportiert werden können.

Ein bekanntes System hängt die Geräte über eine Schwalbenschwanzhalterung und entsprechende Gegenstücke entweder an der Wand oder auch an Deckenarmen zur Befestigung von oben her in die Schwalbenschwanzaufnahme ein. Zum Transport der Geräte dient ein Hubfahrgestell mit einer höhenbeweglichen Plattform. Diese greift unter den Boden des Gerätes und kann dann durch Höhenverstellung das Ein- und Aushängen bewirken. Während des Transports ist das Gerät auf der Plattform praktisch ungesichert gegen Abrutschen abgestellt. Im eingehängten Zustand kann es durch unbeabsichtigt angreifende senkrechte Kräfte, wie es bei der Bewegung des Schwenkarmes, an dem das Gerät angehängt ist, vorkommen kann, zum Aushängen mit allen möglichen Folgen kommen. (Dräger-Prospekt 5222.3, Nov. 1977).

In der DE-B1-2 812 074 ist eine Einrichtung zur verriegelten Aufnahme eines Gerätes, in diesem Falle einer Patientenlagerungsplatte, am Einsatzort, sowie zu dessen verriegeltem Transport beschrieben. Dabei soll die Verriegelung von einem Tischgestell auf ein Fahrgestell und zurück bei allen möglichen Kipplagen des Gerätes automatisch erfolgen. Dazu sind Kupplungen vorgesehen, welche mindestens zwei, in jeweils eine stabile Endlage selbsttätig schwenkbare federbelastete Klinken enthalten. Durch das Herausfahren des jeweiligen anzukoppelnden Gestells werden die Klinken in ihre koppelnde, durch Anschläge begrenzte Endlage übergeführt. Dabei greifen die Klinken mit einem Haken in entsprechende Kupplungsplatten an den Gestellen ein.

Aufgabe der Erfindung ist ein Geräteaufnahmesystem der Medizintechnik, dessen Aufnahmeeinrichtungen den einfachen Zusammenbau und -halt der Geräteanordnungen sowie problemlosen Transport der Geräte sichern.

Die Lösung der Aufgabe erfolgt gemäß dem Kennzeichen des Anspruchs 1. Vorteilhafte Ausbildungen sind in den Ansprüchen 2 bis 4 beschrieben.

Der Geräterahmen, der das Gerät trägt, dient als Zwischenstück, mit dem die Einfügung des Gerätes in die Anordnung und auch dessen Transport, z. B. zum Austauschen, Reinigen und Überwachen gesichert möglich ist.

Der Geräterahmen mit dem Gerät wird auf der Hubwagenplatte des Hubwagens transportiert und auf die Rahmenaufnahme übergeschoben. Die Riegel, in die entsprechende Endstellung geschoben, verriegeln dabei jeweils den Geräterahmen in einfacher Weise entweder mit der Rahmenaufnahme oder mit der Hubwagenplatte. Die Sperren, die bei den Bewegungen der Teile zueinander immer in die vorgesehenen Stellungen geschoben werden, sichern dabei die Riegelstellung. Danach kann der Geräterahmen erst dann auf der Rahmenaufnahme entriegelt werden, wenn die Hubwagenplatte eingeschoben oder umgekehrt, wenn die Rahmenaufnahme unter den von der Hubwagenplatte getragenen Geräterahmen eingeführt wurde.

Es ist in jedem Fall sichergestellt, daß immer nur ein definierter Zustand, d. h. entweder die Verriegelung an der Hubwagenplatte oder die Verriegelung an der Rahmenaufnahme möglich ist.

Ein Ausführungsbeispiel der Erfindung ist in der Zeichnung dargestellt und wird im folgenden beschrieben. Es zeigen

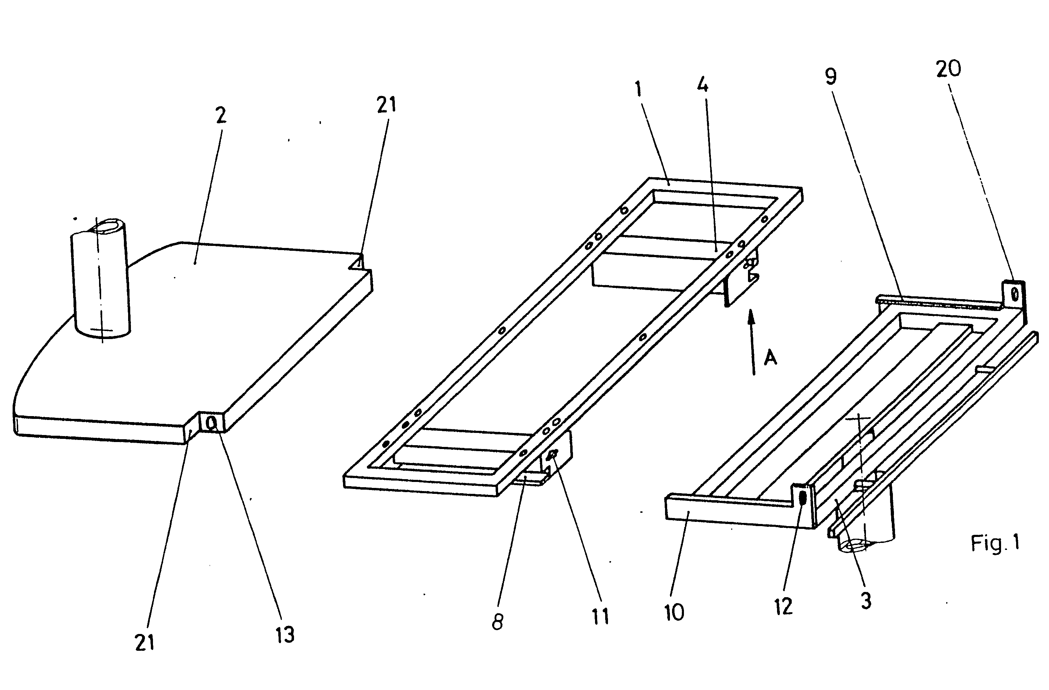

Figur 1 die Elemente des Geräteaufnahmesystems,

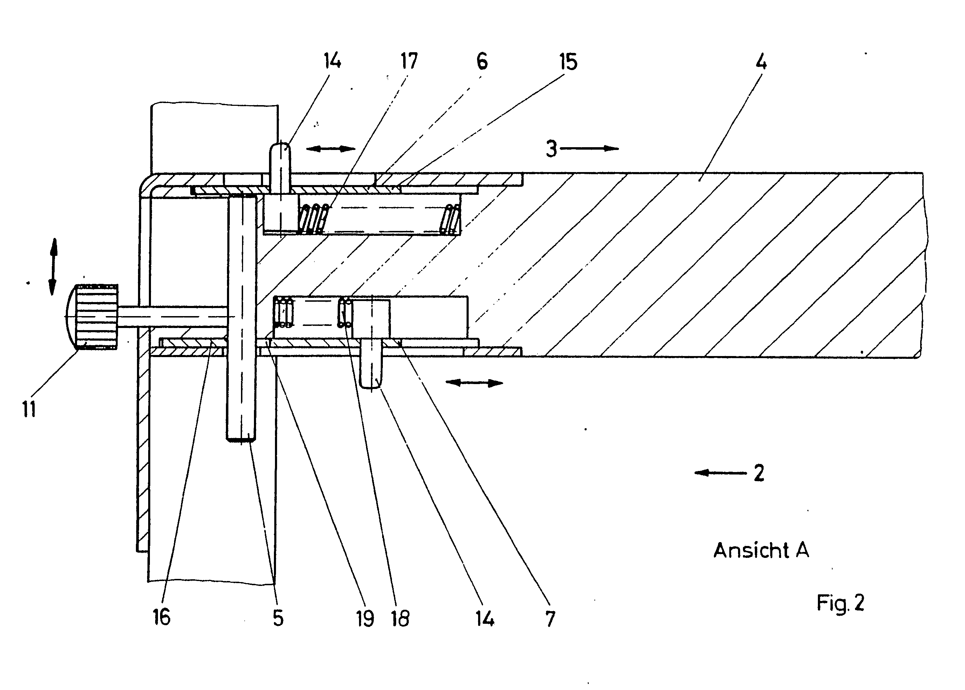

Figur 2 die Verriegelung mit den Sperren in Unteransicht A des Geräteaufnahmerahmens.

Das Geräteaufnahmesystem besteht aus einem Geräterahmen 1 zum Tragen des Gerätes, z. B. eines Narkosegerätes, einer Rahmenaufnahme 2 und einer Hubwagenplatte 3.

Mit dem Geräteaufnahmesystem werden die Geräte, die auf dem Geräterahmen 1 befestigt sind, zu ihrem Einsatzort im OP oder am Bett des Patienten oder von dort zur Reinigung oder Überprüfung transportiert.

Daraus ergeben sich die Betriebszustände :

a) Für den Transport ist der Geräterahmen 1, der das Gerät trägt, mit der Hubwagenplatte 3 verbunden.

b) Während des Betriebes ist der Geräterahmen 1 zusammen mit dem Gerät mit der Rahmenaufnahme 2 verbunden.

Riegel 5 und Sperren 6, 7 stellen sicher, daß sich der Geräterahmen 1 nicht ungewollt von der Hubwagenplatte 3 oder von der Rahmenaufnahme 2 lösen kann.

Der Geräterahmen 1 besitzt Führungsleisten 4, die außen mit Führungsnuten 8 versehen sind, in die Schienen 9 von Führungsblechen 10 der Hubwagenplatte 3 einfahren. Die Führungsleisten 4 enthalten an dem Einfahrende für die Hubwagenplatte 3 je einen quergeführten, über einen Griff 11 in eine äußere und eine innere Endstellung verschiebbaren Riegel 5. In der äußeren Endstellung greift dieser in eine Öffnung 12 der Führungsbleche 10 und in der inneren Endstellung in eine Bohrung 13 der Rahmenaufnahme 2 ein und verriegelt den Geräterahmen 1 dort gegen ein ungewolltes Herunterrutschen.

Zur Sicherung der jeweiligen Endstellung der Riegel 5 besitzen die Führungsleisten 4 außen eine Sperre 6 und innen eine Sperre 7. Diese bestehen je aus einem federbelasteten Mitnahmebolzen 14, die außen ein Sperrblech 15 und innen ein Sperrblech 16 in Einschubrichtung gegen die Federn 17, 18 bewegen. Das Sperrblech 15 sperrt mit entspannter Feder 17 den Riegel 5 in innerer Endstellung, das Sperrblech 16 mit entspannter Feder 18 den Riegel 5 in äußerer Endstellung. Mit gespannter Feder 17 ist der Weg für den Riegel 5 in die äußere Endstellung und mit gespannter Feder 18 durch ein Loch 19 im Sperrblech 16 in die innere Endstellung frei.

Zur Aufnahme des Geräterahmens 1 mit dem Gerät wird die Hubwagenplatte 3 mit dem Hubwagen in die Führungsleiten 4 eingefahren. Im eingefahrenen Zustand drückt ein Lappen 20 an den Führungsblechen 10 die seitlich aus den Führungsleisten 4 herausragenden Mitnahmebolzen 14 der Sperren 6 gegen die Feder 17 nach hinten und gibt damit die Riegel 5 in die äußere Endstellung und damit in die Öffnungen 12 der Lappen 20 frei. Die Riegel 5 werden über den Griff 11 zur Verriegelung in die äußere Endstellung geschoben.

Wenn der Geräterahmen 1 dabei gleichzeitig von der Rahmenaufnahme 2 abgehoben werden soll, auf der er sich, geführt über seine Führungsleisten 4, befindet, so ist dies durch ein geringfügiges Anheben der Hubwagenplatte 3 möglich. Die Verriegelung über die Riegel 5 zwischen dem Geräterahmen 1 und der Rahmenaufnahme 2 wurde gleichzeitig mit der neuen Verriegelung mit der Hubwagenplatte 3 aufgehoben. Der Riegel 5 gab dabei die Bohrung 13 in der Rahmenaufnahme 2 frei.

Die Bewegung der inneren Sperren 7 zur Verriegelung der eingefahrenen Rahmenaufnahme 2 mittels der Riegel 5 in der inneren Endstellung erfolgt über die von Ausnehmungen 21 geschobenen inneren Mitnahmebolzen 14 gegen die Federn 18. Zum Verriegeln treten die Riegel 5 dabei durch die Löcher 19 in den Sperrblechen 16 hindurch und greifen in die Bohrungen 13 ein.

Es ist sichergestellt, daß sowohl im
Betriebszustand 1. für den Transport
Betriebszustand 2. während des Betriebes
der Geräterahmen 1 jeweils gesperrt verriegelt ist.

## Patentansprüche

1. Geräteaufnahmesystem der Medizintechnik mit Einrichtungen zur verriegelten Aufnahme vom Gerät am Einsatzort sowie zum verriegelten Transport gekennzeichnet durch
a) einen Geräterahmen (1) mit daran befestigtem Gerät, der Führungsleisten (4) mit Führungsnuten (8) aufweist,
b) eine Rahmenaufnahme (2), die zur Aufnahme zwischen den Führungsleisten (4) geführt ist und den Geräterahmen (1) trägt,
c) eine Hubwagenplatte (3), die in die Führungsnuten (8) einschiebbar ist und den Geräterahmen (1) nach dessen Abnahme von der Rahmenaufnahme (2) zum Transport trägt,
d) Riegel (5), die in einer inneren oder außeren Endstellung den Geräterahmen (1) entweder mit der Rahmenaufnahme (2) oder mit der Hubwagenplatte (3) verriegeln und
e) Sperren (6, 7), die die Riegel (5) in der inneren oder äußeren Endstellung halten und durch die Bewegung des Geräterahmens (1) relativ zur Rahmenaufnahme (2) bzw. zur Hubwagenplatte (3) betätigt sind.

2. Geräteaufnahmesystem der Medizintechnik nach Anspruch 1, dadurch gekennzeichnet, daß die in den Führungsleisten (4) quer zur Einschubrichtung geführten, mit einem Griff (11) versehenen Riegel (5) in ihrer inneren Endstellung in Bohrungen (13) der Rahmenaufnahme (2) und ihrer äußeren Endstellung in Öffnungen (12) in Lappen (20) von Führungsblechen (10) der Hubwagenplatte (3) eingreifen.

3. Geräteaufnahmesystem der Medizintechnik nach einem der Ansprüche 1 oder 2, dadurch gekennzeichnet, daß eine erste Sperre (6) außen an jeder der Führungsleisten (4) vorgesehen ist, die aus einem mit einer Feder (17) gegen die Einschubrichtung der Hubwagenplatte (3) belasteten Mitnahmebolzen (14) mit einem Sperrblech (15) besteht.

4. Geräteaufnahmesystem der Medizintechnik nach einem der Ansprüche 1 oder 2, dadurch gekennzeichnet, daß eine zweite Sperre (7) innen an jeder der Führungsleisten (4) vorgesehen ist, die aus einem mit einer Feder (18) gegen die Einschubrichtung der Rahmenaufnahme (2) belasteten Mitnahmebolzen (14) mit einem Sperrblech (16) besteht.

## Claims

1. Apparatus-receiving system for the medical field, having fittings for conforming with the apparatus arrangement, characterised in that an apparatus frame (1), with apparatus secured thereto in the apparatus arrangement is supported on a frame receiver (2) guided between guide strips (4), and, after its removal from said receiver for transportation, is supported on a lifting truck plate (3) pushed for this purpose into guide grooves (8) of the guide strips (4), said frame being locked together with either the frame receiver (2) or the lifting truck plate (3) by means of a locking bar (5), which is held in an inner or outer end position by means of stops (6, 7) which are actuated by the movements.

2. Apparatus-receiving system for the medical field according to claim 1, characterised in that the locking bars (5), which are driven in the guide strips (4) transverse to the push-in direction and which are provided with a handle (11), engage, when in their inner end position, into bore holes

(13) of the frame receiver (2), and, when in their outer end position, into openings (12) in tabs (20) of guide plates (10) of the lifting truck plate (3).

3. Apparatus-receiving system for the medical field according to claim 1 and 2, characterised in that the stop (6), which is on the outside of the guide strips (4), consists of a carrier bolt (14), which is loaded with a spring (17) against the push-in direction of the lifting truck plate (3), together with a stop sheet (15).

4. Apparatus-receiving system of medical technology according to claim 1 and 2, characterised in that the stop (7), which is on the inside of the guide strips (4), consists of a carrier bolt (14), which is loaded with a spring (18) against the push-in direction of the frame receiver (2), together with a stop sheet (16).

**Revendications**

1. Système de réception d'appareils médicaux avec des mécanismes pour le verrouillage de l'appareil sur le lieu de montage ainsi que pour le transport, caractérisé par :

a) un cadre d'appareil (1) avec l'appareil fixé dessus et qui présente des barres de guidage (4) avec des rainures de guidage (8),

b) un récepteur de cadre (2) qui est introduit entre les barres de guidage (4) pour la réception et qui porte le cadre d'appareil (1),

c) une plaque de chariot de levage (3) qui est apte à être introduite dans les rainures de guidage (8) et qui porte le cadre d'appareil (1) après son enlèvement du récepteur de cadre (2) en vue du transport,

d) des verrous (5) qui se bloquent dans une position extrême intérieure ou extérieure du cadre d'appareil (1) soit avec le récepteur de cadre (2) soit avec la plaque du chariot de levage (3), et

e) des arrêts (6, 7) qui arrêtent les verrous (5) dans la position extrême intérieure ou extérieure et qui sont actionnés par le déplacement du cadre de l'appareil (1) par rapport au récepteur de cadre (2) ou selon le cas le plateau du chariot de levage (3).

2. Système de réception d'appareils médicaux selon la revendication 1, caractérisé en ce que les verrous (5) munis de manette (11) et qui sont logés dans les barres de guidage (4) perpendiculairement à la direction d'introduction, s'engagent dans leur position extrême intérieure dans des trous (13) du récepteur de cadre (2) et dans leur position extrême extérieure dans des ouvertures (12) ménagées dans des pattes (20) de plaques de guidage (10) du plateau du chariot de levage (3).

3. Système de réception d'appareils médicaux selon l'une des revendications 1 ou 2, caractérisé en ce qu'un premier arrêt (6) est prévu extérieurement sur chacune des barres de guidage (4), et en ce que cet arrêt (6) est formé d'un tourillon d'accrochage (14), avec une plaque de verrouillage (15), chargé par un ressort (17) dans la direction contraire à celle d'introduction du plateau du chariot de levage (3).

4. Système de réception d'appareils médicaux selon l'une des revendications 1 ou 2, caractérisé en ce qu'un second arrêt (7) est prévu à l'intérieur sur chaque barre de guidage (4), et en ce que ce second arrêt (7) est formé d'un tourillon d'accrochage (14), avec une plaque de verrouillage (16), chargé par un ressort (18) dans la direction opposée à celle d'introduction du récepteur de cadre (2).

Fig. 1

Ansicht A

Fig. 2